(19) Europäisches Patentamt
European Patent Office
Office européen des brevets

(11) **EP 2 400 463 A1**

(12) **EUROPEAN PATENT APPLICATION**
published in accordance with Art. 153(4) EPC

(43) Date of publication:
**28.12.2011 Bulletin 2011/52**

(21) Application number: **11755232.3**

(22) Date of filing: **23.03.2011**

(51) Int Cl.:
**G06T 15/08** (2011.01) **A61B 5/055** (2006.01)
**A61B 6/03** (2006.01) **A61B 19/00** (2006.01)
**G06T 1/00** (2006.01) **G09B 9/00** (2006.01)

(86) International application number:
**PCT/JP2011/001699**

(87) International publication number:
**WO 2011/118208 (29.09.2011 Gazette 2011/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.03.2010 JP 2010067610**

(71) Applicant: **Panasonic Corporation**
**Kadoma-shi**
**Osaka 571-8501 (JP)**

(72) Inventors:
• **IMANAKA, Ryoichi**
**(JP)**
• **KOHYAMA, Tsuyoshi**
**(JP)**
• **IMANISHI, Keiho**
**(JP)**

(74) Representative: **Eisenführ, Speiser & Partner**
**Postfach 10 60 78**
**28060 Bremen (DE)**

(54) **CUTTING SIMULATION DEVICE**

(57) This resection simulation apparatus comprises a tomographic image information acquisition section (6), a memory (9) that is connected to this tomographic image information acquisition section (6), a volume rendering computer (13) that is connected to this memory (9), a display (2) that displays the computation result of this volume rendering computer (13), and an input section (4) that performs resection designation with respect to a display object displayed on this display (2). The volume rendering computer (13) samples voxel information in a direction perpendicular to the line of sight from the voxel information stored in at least the memory (9), and a depth detector (15) measures the ray casting scan distances for all points found in movement over the resection designated points.

FIG. 2

EP 2 400 463 A1

**Description**

TECHNICAL FIELDF

The present invention relates to a resection simulation apparatus used when a medical practitioner performs simulated surgery.

BACKGROUND ART

A resection simulation apparatus that allows simulated surgery to be performed is used so that better surgery can be performed in a medical facility.

A conventional resection simulation apparatus of this type comprised a tomographic image information acquisition section, a memory that is connected to this tomographic image information acquisition section, a volume rendering computer that is connected to this memory, a display that displays the computation result of this volume rendering computer, and an input section that issues resection instructions with respect to a display object displayed on this display.

With the above constitution, there is one apparatus with which, rather than displaying voxel labels on a display (two-dimensional display) and having the input section issue resection instructions with respect to the voxel label display object, the display object is displayed in 3D, and resection instructions are issued for a 3D display object (see Patent Literature 1, for example).

CITATION LIST

PATENT LITERATURE

Patent Literature 1: Japanese Laid-Open Patent Application 5-123327

SUMMARY

A problem encountered with the conventional constitution discussed above was the difficulty of performing a good surgical simulation.

Specifically, when voxel labels are displayed on a display (two-dimensional display) and resection instructions are issued using an input section on a display object of these voxel labels, in actual practice there may be a discrepancy in the depth direction (Z direction) in this two-dimensional display. If a plurality of display objects are adjacent here, and if the resection instructions of the input section extend to these adjacent display objects, a state in which the resection goes all the way to an unintended display object may end up being displayed on the display. Therefore, it was difficult to conduct a good surgical simulation with a two-dimensional display such as this.

Taking the above-mentioned problem with two-dimensional display into account, as disclosed in the above publication, when a display object is displayed in 3D (three-dimensionally) on a display and resection instructions are issued for this 3D display object, there is a difference in the depth direction (Z direction) of adjacent display objects. Thus, as discussed above, there is no accidental resection instruction for a plurality of adjacent display objects for which there is a difference in the depth direction.

However, to issue resection instructions while looking at this 3D display, the input section must be moved three-dimensionally just as in actual surgery. This is something that is exceedingly difficult for anyone but a skilled surgeon. As a result, it once again is difficult to carry out a good surgical simulation.

In view of this, it is an object of the present invention to provide a resection simulation apparatus with which a good surgical simulation can be performed.

SOLUTION TO PROBLEM

To achieve this object, the resection simulation apparatus of the present invention comprises a tomographic image information acquisition section, a memory, a volume rendering computer, a display, an input section, and a depth detector. The tomographic image information acquisition section acquires tomographic image information. The memory is connected to the tomographic image information acquisition section and stores voxel information for the tomographic image information. The volume rendering computer is connected to the memory and samples voxel information in a direction perpendicular to the sight line on the basis of the voxel information. The display displays the computation result of the volume rendering computer. The input section inputs resection instructions with respect to a displayed object that is displayed on the display. The depth detector measures the ray casting scan distance for all points found during the movement of the input section over points designated for resection by the input section.

Here, the voxel labels are information for showing the result of resection instructions or other such processing performed by the user, and has the same configuration as the voxel information. In the initial state, this is set to a value decided to be the voxel label (such as "1"). With this resection simulation apparatus, the volume rendering computer displays information about a plurality of slices which are perpendicular to the line of sight and are regularly spaced in the Z direction, on the display as a three-dimensional image, on the basis of the voxel information, etc., stored in the memory.

Consequently, if there is actually a difference in the positions in the depth direction (Z direction) on the display, then even if a resection instruction inputted from the input section extends to both of them, adjacent display objects will not be displayed in a state of having been accidentally resected. As a result, a good surgical simulation can be performed.

ADVANTAGEOUS EFFECTS

Because of the above constitution of the present invention, adjacent display objects for which there is an actual difference in the positions in the depth direction (Z direction) are prevented from being displayed in a state of having been accidentally resected, so a good surgical simulation can be performed.

BRIEF DESCRIPTION OF DRAWINGS

FIG. 1 is an oblique view of the configuration of the resection simulation apparatus pertaining to Embodiment 1 of the present invention;
FIG. 2 is a control block diagram of the resection simulation apparatus in FIG. 1;
FIGS. 3 (a) and 3 (b) are operation flowcharts for the resection simulation apparatus in FIG. 1;
FIG. 4 is a concept diagram illustrating the operation of the resection simulation apparatus in FIG. 1; and
FIG. 5 is a diagram of an example of the image displayed on the display of the resection simulation apparatus in FIG. 1.

DESCRIPTION OF EMBODIMENTS

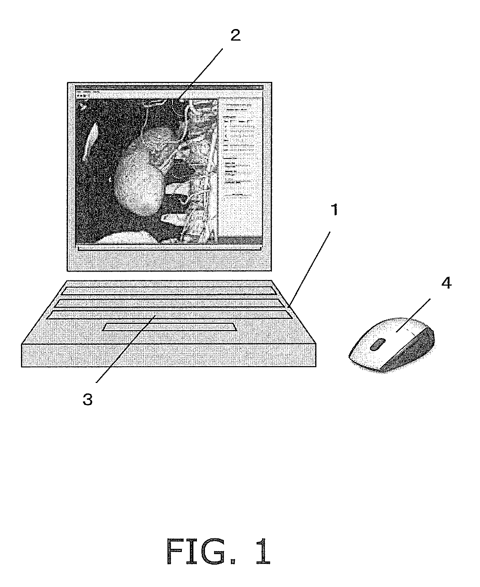

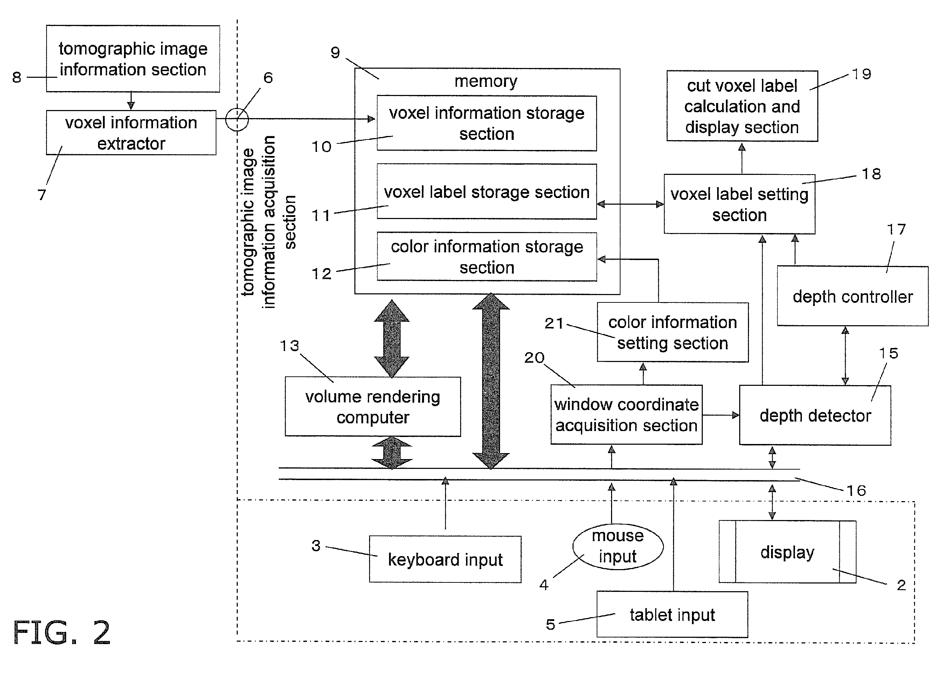

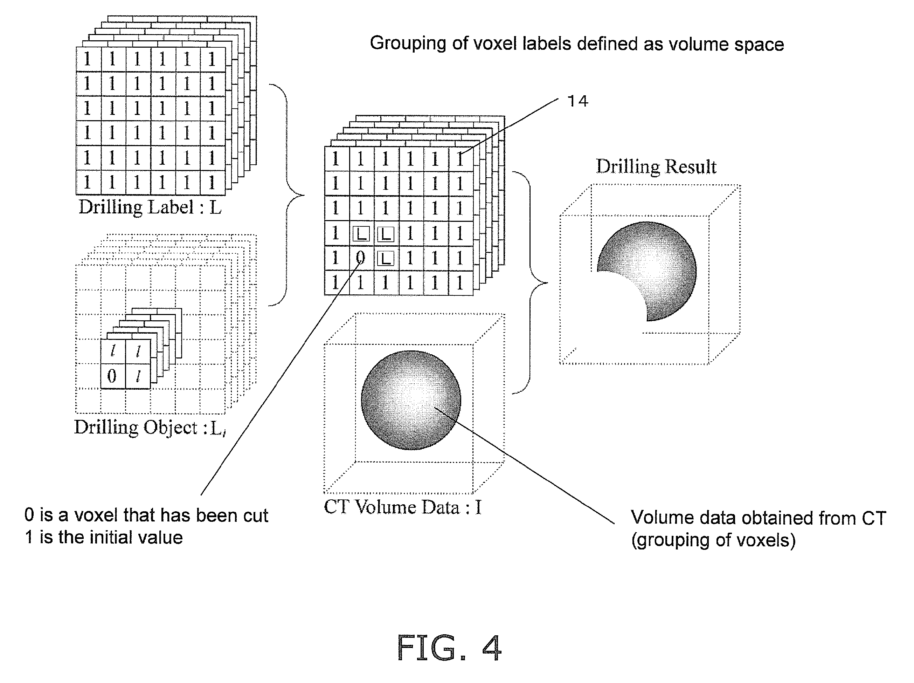

The resection simulation apparatus pertaining to an embodiment of the present invention will now be described in detail along with the drawings.
The personal computer 1 (resection simulation apparatus) shown in FIG. 1 comprises a display 2, an input section (keyboard input 3, mouse input 4, and tablet input 5) (see FIG. 2). The keyboard input 3 is a keyboard type. The mouse input 4 is a mouse type. The tablet input 5 is a tablet type.
FIG. 2 is a diagram of the control blocks formed in the personal computer 1.
The tomographic image information acquisition section 6 shown in FIG. 2 is connected via a voxel information extractor 7 to a tomographic image information section 8. That is, with the tomographic image information section 8, tomographic image information is supplied from a CT or MRI, and this tomographic image information is extracted as voxel information by the voxel information extractor 7. This voxel information is stored in a voxel information storage section 10 of a memory 9 via the tomographic image information acquisition section 6.
The memory 9 is provided inside the personal computer 1, and comprises a voxel label storage section 11 and a color information storage section 12 in addition to the voxel information storage section 10.
The memory 9 is also connected to a volume rendering computer 13.
The volume rendering computer 13 obtains information for a plurality of slices which are perpendicular to the line of sight and are regularly spaced in the Z direction, as shown in FIG. 4, on the basis of the voxel information stored in the voxel information storage section 10 of the memory 9, the voxel labels stored in the voxel label storage section 11, and the color information stored in the color information storage section 12. The volume rendering computer 13 also displays this computation result as a three-dimensional image on the display 2. The volume rendering computer 13 is connected to a depth detector 15 that measures the ray casting scan distance (discussed below) via a bus 16.
The depth detector 15 is connected to a depth controller 17 and a voxel label setting section 18. The voxel label setting section 18 is connected to the voxel label storage section 11 and a resection voxel label calculation and display section 19.
In addition to what is mentioned above, the bus 16 is connected to the color information storage section 12 and a window coordinate acquisition section 20. The window coordinate acquisition section 20 is connected to the depth detector 15 and a color information setting section 21. The color information setting section 21 is connected to the color information storage section 12.

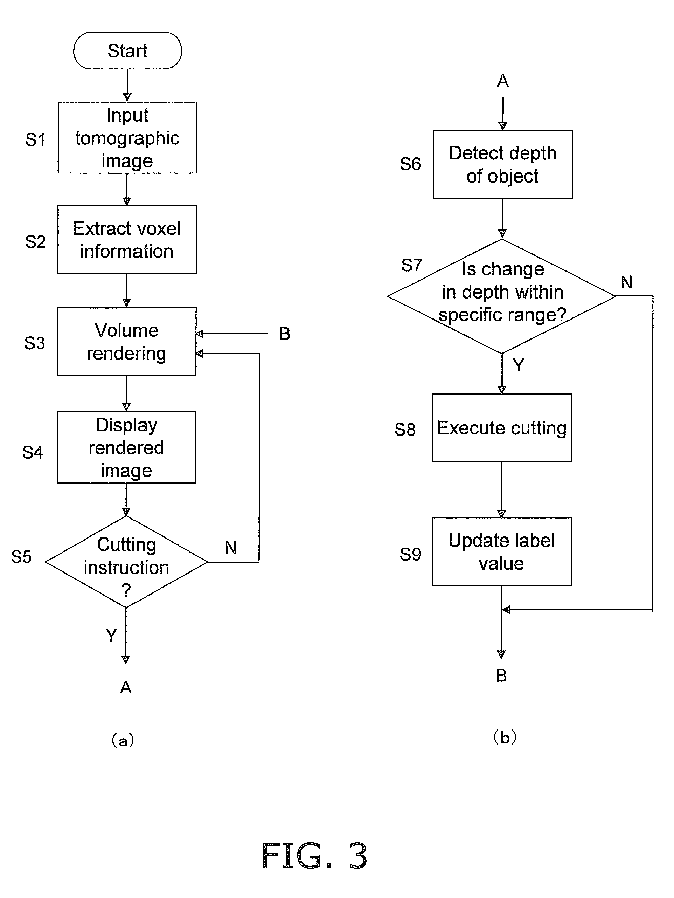

FIGS. 3 (a) and 3 (b) are control flowcharts illustrating the operation of the resection simulation apparatus of this embodiment.
First, in step S1, as mentioned above, tomographic image information is obtained from the tomographic image information section 8, and this is supplied to the voxel information extractor 7.
Next, in S2, voxel information is extracted by the voxel information extractor 7. The voxel information is stored via the tomographic image information acquisition section 6 in the voxel information storage section 10 of the memory 9. The voxel information stored in the voxel information storage section 10 is information about the points that make up I (x, y, z, $\alpha$). I here is brightness information for said points, x, y, and z are coordinate points, and $\alpha$ is transparency information.
Next, in S3, the volume rendering computer 13 calculates information about a specific number of slices that are perpendicular to the line of sight and are regularly spaced, on the basis of voxel information stored in the voxel information storage section 10, and acquires a slice information group. The slice information group is also stored, at least temporarily, in the volume rendering computer 13.
The above-mentioned "information about slices perpendicular to the line of sight" means a plane that is at a right angle to the line of sight. For instance, when the display 2 is set up vertically and it is viewed in a state in which it and the viewer's head are horizontal, the slice information is constituted by a plane that is perpendicular to the line of sight.
The information about a plurality of slices thus obtained includes information for the points constituted by I (x, y, z, $\alpha$), as mentioned above. This slice information comprises a plurality of voxel labels 14 laid out in the Z direction as shown in FIG 4, for example. The grouping of voxel labels 14 shown in FIG 4 is stored in the voxel label storage section 11, for example.

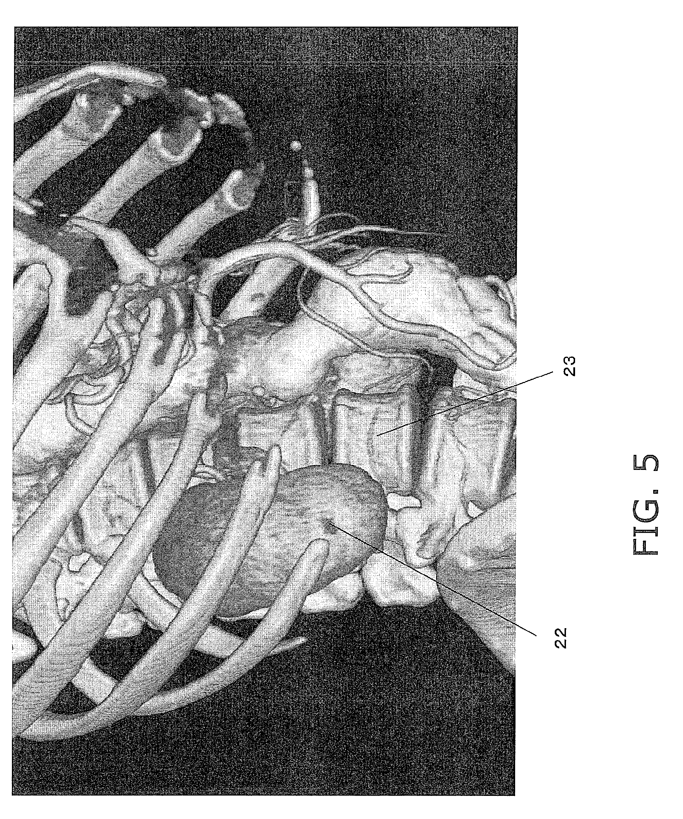

Next, in S4, as shown in FIG. 5, a rendering image is displayed on the display 2. On the display 2 at this point a resection object is selected with the mouse input 4, and this is displayed as shown in FIG. 5. That is, 22 in FIG. 5 is a kidney, and 23 is a backbone. In this embodiment, we will assume that a simulation of surgery on the kidney 22 is to be performed.

As can be seen from FIG. 5, with the display 2 in this embodiment, even though the kidney 22 is actually in front of the backbone 23, the two also appear to be adjacent in planar view. In this embodiment, a slice image that includes the kidney 22 and the backbone 23 has information about the points constituted by I $(x, y, z, \alpha)$. Accordingly, as will be discussed below, in a simulation, when the user wants to resect the kidney 22, which is in front on the screen of the display 2, control must be performed as follows so that the backbone 23 is not resected at the same time on the screen.

In S5, a resection instruction is issue. In this embodiment, a resection instruction is issued by using the mouse input 4. The input section may be either the keyboard input 3, the mouse input 4, or the tablet input 5.

More specifically, when the mouse input 4 is moved horizontally over a desktop, the cursor indicated on the display 2 moves up and down, or to the left and right, over the kidney 22.

The left-right or up-down movement of the mouse input 4 here is detected by the window coordinate acquisition section 20. This information is transmitted through the depth detector 15 to the voxel label setting section 18 and the voxel label storage section 11. Consequently, resection is performed that takes into account the positions of the kidney 22 and the backbone 23 in the Z direction.

More specifically, the volume rendering computer 13 samples voxel information at constant intervals in a direction perpendicular to the line of sight (this is called ray casting). The volume rendering computer 13 then calculates the proportional change in the ray casting scan distance measured by the depth detector 15 for all the points found during the mouse movement.

More specifically, the ray casting scan distances d measured by the depth detector 15 are tabulated, and the gradient $\nabla d$ thereof is calculated. The gradient $\nabla d$ is compared with a threshold T to determine whether or not resection needs to be executed. For example, if a gradient $\nabla d_i$ at a resection point $p_i$ is at least a threshold $T_i$, the resection point is deemed invalid, and resection is not performed.

As to the threshold T, the threshold $T_i$ is determined on the basis of a multiple coefficient m and gradient average for n number of resection points in the immediate vicinity for each resection processing.

$$\mathbf{T}_i = m\left( \sum_{k=i-1-n}^{k=i-1} \nabla \mathbf{d}_k \right) / n$$

The multiple coefficient m and the resection point n can be suitably set according to the image being processed, with their numerical values being about 5 for m and 10 for n, for example.

Erroneous resection can be avoided even if a resection point is detected at an abruptly lower depth due to a mistake in user operation. As a result, resection is performed only in smooth changes in depth.

Thus, in this embodiment, the gradient $\nabla d$ and the threshold $T_i$ calculated on the basis of the multiple coefficient m and the gradient average for n number of resection points in the immediate vicinity are compared, and result is used as the proportional change, and whether or not to perform resection can thereby be determined.

How the proportional change is calculated is not limited to what is given in this embodiment, and any calculation formula may be used as long as it allows the gradient change state to be confirmed.

Also, suitably varying the threshold T according to the characteristics of the organ that is to be resected further increases the accuracy at which erroneous resection is avoided.

In the resection processing discussed above, a point having a proportional change over a specific threshold is considered to be an invalid resection point, and the depth controller 17 issues an instruction to the voxel label setting section 18. Consequently, updating of the voxel labels is halted, and resection is not carried out. Thus, erroneous resection can be avoided when the depth detector 15 has detected a resection point whose depth position changes abruptly due to operational error by the user.

Here, the phrase "resection is performed" means that the voxel label setting section 18 updates the voxel labels and stores them in the voxel label storage section 11. That is, when resection is not performed, the voxel labels do not change.

Therefore, even if the mouse input 4 is slid over the kidney 22, the system avoids accidentally resecting the backbone 23 located deeper to the inside. In this case, an image in which just the kidney 22 has been resected is displayed according to how many times the mouse input 4 has been slid to the left and right or up and down.

A state in which the kidney 22 is resected can be confirmed by the fact that the color of the kidney 22 changes when information from the window coordinate acquisition section 20 is sent through the color information setting section 21 to the color information storage section 12. The "color information setting section 21" here means a converter that employs what is known as a look-up table. That is, with the personal computer 1 in this embodiment, as discussed above, there is information about the points constituted by I (x, y, z, α), and different color information and brightness information are set ahead of time by the color information setting section 21 for the surface and the interior of the kidney 22. Consequently, if user operation indicates resection from the surface, the color of the resected portion will be displayed as being clearly different from the surrounding color according to the degree of this resection.

The above-mentioned state is the state in steps S6, S7, and S8, and in S9 the voxel information at the resection site is updated.

FIG. 4 shows this state, and shows a state in which most of the voxel labels 14 on the outer surface are "1," that is, it shows the measured surface state of the kidney 22. In FIG. 4, the "0" portion indicates a voxel that has been resected. "L" is used to make the state of the resection voxel and its surroundings easier to recognize with color information. For example, if the "1" is a bright reddish-brown color, and "0" is red, for "L" an intermediate color from bright reddish-brown to red is selected for the boundaries. This allows the actual progress of the resection to be expressed in a way that is intuitively grasped (combining the two graphics on the left in FIG. 4 (drilling label and drilling object) forms an image that shows how the resection is progressing). Also, the resection state on the right (drilling result) is formed on the basis of the two graphics in the middle in FIG. 4.

As discussed above, with this embodiment, the volume rendering computer 13 samples voxel information at regular intervals in a direction perpendicular to the line of sight. The proportional change in the ray casting scan distances calculated by the depth detector 15 is then calculated for all the points found during the mouse movement.

Here, the depth controller 17 outputs an instruction to the voxel label setting section 18, using points having a proportional change over a specific threshold as invalid resection points, for the calculated proportional change, and performs control such that the updating of the voxel labels is halted and no resection is performed.

Consequently, as long as what is being resected actually has a difference in its position in the depth direction (Z direction) on the display 2, even if the resection instruction from the mouse input 4 extends to both of them, it will be possible to avoid the accidental resection of adjacent display objects. As a result, good surgical simulation can be carried out.

In this embodiment, for example, the start and end of resection is switched by clicking the mouse button on and off, and the user drags the mouse with the mouse button clicked on, which allows the resection of the intended region to be carried out continuously.

Also, in this embodiment, the timing at which the memory 9 is updated can be set to when the mouse button is off. When the user starts dragging the mouse while holding down the mouse button, just the memory of the volume rendering computer 13 is updated, which provides the user with a visually interactive resection function. Here, volume labels during work are temporarily stored, without updating the memory 9. When the user releases the button, the memory content that had been temporarily stored is reflected in the memory 9. Adding control such as this allows a display in which the object has been resected only down to a specific depth from its surface in a single drag operation by the user, so display of an excessively resected state is prevented.

Also, in this embodiment, the voxel labels are the same size as the initial voxel information, but to express more precise resection, voxel labels may be produced in a smaller size. With this method, the voxel information is not directly edited, and the voxel labels are given time information, which makes possible operations such as undo and redo.

Also, in this embodiment, surgical simulation can be performed merely by moving the mouse input 4 in a planar fashion, without issuing a resection instruction while looking at a 3D display. Thus, the surgical simulation is favorable from this standpoint as well.

Other Embodiments

In the above embodiment, an example was described in which the brightness information and color information of a display object were both varied in the voxel labels 14 for which a resection instruction was issued with the mouse input 4, but the present invention is not limited to this. For example, just the brightness information or color information of the display object may be varied.

Furthermore, in the above embodiment, the amount of resection (volume) with the mouse input 4 may be displayed on the display 2 as the output of the resection voxel label calculation and display section 19 that calculates the volume of the voxels that are resected.

Instead of this, the resection depth with the mouse input 4 may be displayed on the display 2.

Furthermore, a resection simulation may be performed so that the resection operation is reflected by a three-dimensional image even when additionally projecting a two-dimensionally sliced image on a three-dimensional image showing the result of volume rendering, and performing a resection operation on the two-dimensionally sliced image.

Furthermore, voxel information stored in the voxel information storage section 10 may be displayed on the display 2 two-dimensionally or after being converted into a three-dimensional image, and the color information setting section 21 may be provided for changing the color information for the portion designated with the mouse input 4 in the resection object displayed on the display 2. That is, in the resection object displayed on the display 2, for example, a color is intentionally added to the portion that is of interest to a physician, and a grouping of voxel labels 14 in this state is stored in the voxel label storage section 11. Consequently, all of the information to which color has been added is reflected in the display from all the places from which this information was extracted. Thus, this portion of interest can be viewed stereoscopically from all around, and this resection simulation can also be carried out.

Furthermore, the present invention allows for the simulation of endoscopic surgery, in which case the convergence characteristics of a fisheye lens or the like provided to an endoscope may be used as a coordinate conversion table in the volume rendering computer 13.

It is also possible to produce a stereoscopic image by having a plurality of viewpoints, storing in a plurality of memories the output images of the volume rendering computer 13 produced for each viewpoint, and displaying this output successively from the memories. In this case, a liquid crystal glass or the like that is synchronized to the image outputs may be used.

INDUSTRIAL APPLICABILITY

As discussed above, with the present invention, surgical simulation can be performed merely by moving an input section in a planar fashion, without issuing resection instructions while looking at a 3D display, so a benefit is that good surgical simulation can be carried out, which means that the present invention is expected to have broad applicability as a resection simulation apparatus for performing surgery.

REFERENCE SIGNS LIST

1    personal computer (resection simulation apparatus)
2    display
3    keyboard input (input section)
4    mouse input (input section)
5    tablet input (input section)
6    tomographic image information acquisition section
7    voxel information extractor
8    tomographic image information section
9    memory
10    voxel information storage section
11    voxel label storage section
12    color information storage section
13    volume rendering computer
14    voxel label
15    depth detector
16    bus
17    depth controller
18    voxel label setting section
19    resection voxel label calculation and display section
20    window coordinate acquisition section
21    color information setting section
22    kidney

23    backbone

## Claims

1. A resection simulation apparatus, comprising:

   a tomographic image information acquisition section configured to acquire tomographic image information;
   a memory that is connected to the tomographic image information acquisition section and stores voxel information for the tomographic image information;
   a volume rendering computer that is connected to the memory and samples voxel information in a direction perpendicular to the sight line on the basis of the voxel information;
   a display configured to display the computation result of the volume rendering computer;
   an input section configured to input resection instructions with respect to a display object that is displayed on the display; and
   a depth detector configured to measure the ray casting scan distance for all points found during the movement of the input section over points designated for resection by the input section.

2. The resection simulation apparatus according to Claim 1,
   wherein the volume rendering computer halts resection when the depth information detected by the depth detector has a rate of change of at least a specific threshold.

3. The resection simulation apparatus according to Claim 1 or 2,
   wherein brightness information, color information, X, Y, and Z information, and a plurality of voxel labels corresponding to the plurality of voxel information are supplied to the volume rendering computer.

4. The resection simulation apparatus according to Claim 3,
   wherein the volume rendering computer changes the brightness information and/or color information for the portion of the display object in the voxel label designated for resection by the input section.

5. The resection simulation apparatus according to any of Claims 1 to 4,
   wherein the display shows a resection amount designated by the input section.

6. The resection simulation apparatus according to any of Claims 1 to 4,
   wherein the display shows a resection depth designated by the input section.

**7.** The resection simulation apparatus according to any of Claims 1 to 6, wherein the memory has a voxel information storage section configured to store voxel information inputted via the tomographic image information acquisition section, and a voxel label storage section configured to store the voxel label resected by the volume rendering computer.

**8.** The resection simulation apparatus according to Claim 7, wherein the memory further has a color information storage section configured to store color information for each voxel label.

**9.** The resection simulation apparatus according to Claim 7 or 8, wherein the volume rendering computer displays on the display the voxel information stored in the voxel information storage section, and further comprising a color information setting section configured to change the color information of the portion designated for resection by the input section in the display object displayed on the display.

FIG. 1

FIG. 2

EP 2 400 463 A1

**(a)**

```
        ┌─────────┐
        │  Start  │
        └────┬────┘
             ▼
      ┌──────────────┐
S1    │    Input     │
      │ tomographic  │
      │    image     │
      └──────┬───────┘
             ▼
      ┌──────────────┐
S2    │ Extract voxel│
      │ information  │
      └──────┬───────┘
             ▼
      ┌──────────────┐
S3    │    Volume    │◄──── B
      │  rendering   │◄──┐
      └──────┬───────┘   │
             ▼           │
      ┌──────────────┐   │
S4    │   Display    │   │
      │   rendered   │   │
      │    image     │   │
      └──────┬───────┘   │
             ▼           │
          ◇ Cutting ◇ ──N┘
S5     ◇ instruction ◇
          ◇    ?   ◇
             │Y
             ▼
             A
```

**(b)**

```
             A
             ▼
      ┌──────────────┐
S6    │ Detect depth │
      │  of object   │
      └──────┬───────┘
             ▼
       ◇  Is change  ◇
S7     ◇ in depth within ◇ ──N──┐
       ◇ specific range? ◇      │
             │Y                 │
             ▼                  │
      ┌──────────────┐          │
S8    │Execute cutting│         │
      └──────┬───────┘          │
             ▼                  │
      ┌──────────────┐          │
S9    │ Update label │          │
      │    value     │          │
      └──────┬───────┘          │
             │◄─────────────────┘
             ▼
             B
```

# FIG. 3

Grouping of voxel labels defined as volume space

Drilling Label : L

Drilling Object : $L_i$

14

Drilling Result

CT Volume Data : I

0 is a voxel that has been cut
1 is the initial value

Volume data obtained from CT
(grouping of voxels)

FIG. 4

EP 2 400 463 A1

FIG. 5

**INTERNATIONAL SEARCH REPORT**

| International application No. |
| --- |
| PCT/JP2011/001699 |

**A. CLASSIFICATION OF SUBJECT MATTER**
*G06T15/08*(2011.01)i, *A61B5/055*(2006.01)i, *A61B6/03*(2006.01)i, *A61B19/00*
(2006.01)i, *G06T1/00*(2006.01)i, *G09B9/00*(2006.01)i

According to International Patent Classification (IPC) or to both national classification and IPC

**B. FIELDS SEARCHED**

Minimum documentation searched (classification system followed by classification symbols)
G06T15/00-15/87

Documentation searched other than minimum documentation to the extent that such documents are included in the fields searched
| | | | |
| --- | --- | --- | --- |
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| X<br>Y | JP 1-291391 A  (Toshiba Corp.),<br>22 November 1989 (22.11.1989),<br>page 1, lower right column, lines 12 to 15;<br>page 3, lower left column, lines 13 to 19; page 4, upper left column, line 8 to upper right column, line 15; page 5, upper right column, line 20 to lower left column, line 3<br>(Family: none) | 1<br>3-9 |
| Y | JP 5-108840 A  (Toshiba Corp.),<br>30 April 1993 (30.04.1993),<br>paragraphs [0002] to [0003]<br>(Family: none) | 3,4,7-9 |

☒ Further documents are listed in the continuation of Box C.    ☐ See patent family annex.

| | |
| --- | --- |
| *    Special categories of cited documents:<br>"A"  document defining the general state of the art which is not considered to be of particular relevance<br>"E"  earlier application or patent but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |

| Date of the actual completion of the international search<br>   11 April, 2011 (11.04.11) | Date of mailing of the international search report<br>   19 April, 2011 (19.04.11) |
| --- | --- |
| Name and mailing address of the ISA/<br>   Japanese Patent Office | Authorized officer |
| Facsimile No. | Telephone No. |

Form PCT/ISA/210 (second sheet) (July 2009)

**INTERNATIONAL SEARCH REPORT**

International application No.

PCT/JP2011/001699

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 2004-215701 A  (Aloka Co., Ltd.), 05 August 2004 (05.08.2004), paragraphs [0001], [0052] (Family: none) | 3,4,8 |
| Y | JP 2-54381 A (Toshiba Corp.), 23 February 1990 (23.02.1990), page 2, upper right column, line 14 to lower left column, line 11; page 5, upper right column, lines 3 to 6; page 5, lower right column, lines 6 to 11; fig. 11 (Family: none) | 5,6 |
| A | JP 64-37678 A  (Toshiba Corp.), 08 February 1989 (08.02.1989), entire text; all drawings (Family: none) | 1-9 |
| A | JP 3-219377 A  (Toshiba Corp.), 26 September 1991 (26.09.1991), entire text; all drawings (Family: none) | 1-9 |

Form PCT/ISA/210 (continuation of second sheet) (July 2009)

**REFERENCES CITED IN THE DESCRIPTION**

**Patent documents cited in the description**

• JP 5123327 A